# EUROPÄISCHE PATENTSCHRIFT

(11) **EP 2 977 157 B1**
(45) Veröffentlichungstag und Bekanntmachung des Hinweises auf die Patenterteilung: **20.02.2019**
(21) Anmeldenummer: 15002018.8
(22) Anmeldetag: 06.07.2015
(51) Int. Cl.: B27M 3/00, G01N 25/00

(54) **VERFAHREN ZUR BEEINFLUSSUNG UND REGELUNG EINES VERLEIMUNGSPROZESSES**
METHOD FOR INFLUENCING AND CONTROLLING A GLUEING PROCESS
PROCÉDÉ DESTINÉ À INFLUENCER ET À RÉGLER UN PROCESSUS DE COLLAGE

(30) Priorität: 23.07.2014 DE 102014214363
(43) Veröffentlichungstag der Anmeldung: 27.01.2016
(73) Patentinhaber: Türmerleim GmbH, 67061 Ludwigshafen (DE)
(72) Erfinder: Althaus, Andy, 68766 Hockenheim (DE); Baun, Willi, 67574 Osthofen (DE); Ernst, Robert, 85135 Titting (DE); Mohr, Claus, 89703 Ulm (DE); Schiller, Johannes, 76857 Ramberg (DE); Weber, Jochen, 78073 Bad Dürrheim (DE); Trippner, Tanguy, 67373 Dudenhofen (DE)
(74) Vertreter: Hörschler, Wolfram Johannes

(56) Entgegenhaltungen:
- WO-A1-92/01540
- WO-A1-2009/071738
- WO-A1-2011/141402
- FRAUNHOFER-INSTITUT FÜR HOLZFORSCHUNG,: QUALITÄTSSICHERUNG BEI DER HOCHFREQUENZVERKLEBUNG VON BRETTSCHICHTHOLZ, [Online] 15040, 31 October 2010 (2010-10-31), pages 5-47, WILHEM-KLAUDITZ-INSTITUT (WKI)

## Beschreibung

### Stand der Technik

Die Erfindung bezieht sich auf ein Verfahren zur Beeinflussung und Regelung eines Verleimungsprozesses, gemäß dem Oberbegriff des Anspruchs 1. Ein solches Verfahren ist aus der folgenden Veröffentlichung bekannt: "Qualitätssicherung bei der Hochfrequenzverklebung von Brettschichtholz", Fraunhofer-Institut für Holzforschung Wilhelm-Klauditz-Institut (WKI), Technische Universität Braunschweig Institut für Nachrichtentechnik (IfN), Technische Universität Hamburg-Harburg, Institut für Hochfrequenztechnik (IHF), März 2010.

Zur Gewährleistung der einwandfreien Funktion des Brettschichtholzes dürfen hierfür nur zugelassene Klebstoffe verwendet werden, die z.B. nach EN 301 und EN 302 geprüft und für diesen Anwendungsbereich speziell anerkannt sein müssen.

Um Brettschichtholz größerer Länge herstellen zu können, muss eine zugfeste Längsverbindung zwischen den einzelnen Brettern einer Lage hergestellt werden. Diese Verbindung wird durch die sogenannte Keilzinkung zum Herstellen endloser Brettlamellen durchgeführt. Für Brettschichtholz wird ca. 80 % Fichtenholz verwendet, obwohl nach den einschlägigen DIN-Normen auch Tanne, Kiefer, Lärche, Douglasie, Western Hemlock und Southern Pine verwendet werden dürfen.

Aus dem am 31.03.2010 veröffentlichten Bericht "Qualitätssicherung bei der Hochfrequenzverklebung von Brettschichtholz" AiF-Nr.: 15040 N / 1 - 3, des Fraunhofer-Institutes für Holzforschung, Wilhelm-Klauditz-Institut (WKI), dem WKI-Forum 2007 "Qualitätssicherung bei der Hochfrequenzverklebung von Brettschichtholz" und dem 11. Kolloquium in Frankfurt vom 22.-23.02.2011 "Qualitätssicherung bei der Hochfrequenzverklebung von Brettschichtholz" (AiF 15040 N / 1 - 3), ist bekannt, dass durch automatisches Ablängen aus dem endlos keilgezinkten Lamellenstrang, Einzellamellen mit bestimmter Länge hergestellt werden. Vor der Beleimung werden die einzelnen Lamellen gehobelt, um so eine optimale Oberflächenbeschaffenheit zu erreichen. Der Leimauftrag erfolgt mit Hilfe spezieller Gießmaschinen in Form von Schnüren oder Vorhängen bei einem Vorschub der Lamellen von bis zu 300 m/min und einer Leimauftragsmenge, die zwischen 180 g/m² und 600 g/m² variieren kann. Danach werden sie entweder mit der Hand oder maschinell in eine Presse eingelegt und mit einem Pressdruck von 0,6 N/mm² bis 0,9 N/mm² bei geraden Teilen und 0,8 N/mm² bis 1,0 N/mm² bei gekrümmten Teilen verpresst. Die sich einstellende Presszeit ist im Wesentlichen abhängig von der im Bauteil bzw. in der Leimfuge vorherrschenden Temperatur sowie des eingesetzten Leimsystems und dessen Mischungsverhältnis. Auch die Umgebungsfeuchte sowie die Umgebungstemperatur haben einen nicht unerheblichen Einfluss. Durch Nutzung der Hochfrequenztechnik lässt sich die benötigte Zeit für die Aushärtung des Leimes wesentlich verkürzen. Dadurch können Bauteile in deutlich kürzeren Takt- bzw. Durchlaufzeiten hergestellt werden.

Weil Holz über eine im Allgemeinen schlechte Wärmeleitfähigkeit verfügt, erfordern Erwärmungsprozesse, bei denen Wärme z.B. über Pressbleche von außen zugeführt wird, entsprechend viel Zeit. Je größer die zu verklebenden Holzdicken sind, umso länger dauert es, bis die erforderliche Wärme dort ankommt, wo sie gebraucht wird. Daraus ergeben sich lange Presszeiten. Außerdem ist eine vollständige Durchwärmung des Gesamtmaterials oft unerwünscht.

Beim Einsatz der Hochfrequenztechnik im Rahmen einer Holzverleimung werden Holz bzw. Trägermaterial und Klebstoff einer kapazitiven oder dielektrischen Erwärmung in einem hochfrequenten Wechselspannungsfeld ausgesetzt. Hierbei geraten die Moleküle in hochfrequente Schwingungen und in Folge innerer Reibung entsteht Wärme. Unter dem Einfluss der elektrischen Wechselfelder richten sich die Moleküle senkrecht zu den Elektroden aus. Mit jedem Richtungswechsel der angelegten Wechselspannung drehen sie sich jeweils um 180°. Dabei entsteht die gewünschte Wärme. Die Häufigkeit dieses Vorgangs richtet sich nach der verwendeten Frequenz.

Eine Klebefuge wird sofort nach dem Anlegen der hochfrequenten Wechselspannung erwärmt. Dies geschieht umso schneller, je größer der Unterschied der dielektrischen Verluste zwischen Leimfuge und dem umgebenden Holz ist. Dieser Vorgang wird auch als selektive Erwärmung bezeichnet. Wasser und andere Moleküle im Leim mit ausgeprägter Dipol-Charakteristik werden umso mehr aus ihrer Ruhelage ausgelenkt, je höher die Spannung an den Elektroden ist. So wird mit zunehmendem zurückgelegtem Weg eine größere Reibungsarbeit geleistet, dementsprechend wächst die Wärmentwicklung im Quadrat zur angelegten Spannung. Wird die angelegte Spannung beispielsweise verdoppelt, so vervierfacht sich die Wärmeentwicklung. Bei der Verarbeitung von Brettschichtholz kommen nahezu ausschließlich Hochfrequenzpressen zum Einsatz, bei denen die Leimfugen senkrecht zu den Kondensatorplatten und parallel zu den Feldlinien ausgerichtet sind. Da hier ein elektrischer Strom fließen kann, spielt die elektrische Leitfähigkeit der Komponenten zum Begünstigen der Wärmeentwicklung ebenfalls eine wichtige Rolle. Auf die Lamellenbreite bezogen ist die geringste Temperatur immer im Randbereich, die höchste dagegen immer in der Lamellenmitte zu erwarten.

Es werden sowohl taktweise als auch kontinuierlich arbeitende Pressensysteme eingesetzt. Bei der taktweisen Verpressung werden die Lamellen aufgestellt, wodurch die Leimfugen senkrecht angeordnet sind. Verschiedene Bauteillängen von Brettschichtholz können in einem Pressvorgang realisiert werden, wobei die Presszeit und somit die Zeit der Einwirkung des hochfrequenten Feldes abhängig von den Schließ- und Öffnungszyklen der Presse ist. Bei einer kontinuierlichen Verpressung (Durchlaufpresse) sind die Leimfugen waagerecht angeordnet und jedes Bauteil je Presse hat die gleiche Länge. Hier wird die Presszeit über die Vorschubgeschwindigkeit des Durchlaufs definiert.

Für eine Hochfrequenzverleimung eignen sich besonders Polykondensationsklebstoffe und Polyvinylacetatleime. Es werden vorzugsweise Kunstharze auf Harnstoff-Formaldehyd, Melamin-Harnstoff-Formaldehyd oder MelaminFormaldehyd-Basis eingesetzt, die bei Temperaturen zwischen 50°C und 90°C deutlich schneller als bei Raumtemperatur abbinden. Diese Leime werden gemeinsam mit Härtern verarbeitet, wobei die Aushärtung durch eine Polykondensation erfolgt und ein Duroplast entsteht.

Bei Leimen auf PVAC Basis handelt es sich im Gegensatz zu den genannten Duroplasten um thermoplastische Leime. Dies bedeutet, dass der Klebstoff während der Erwärmung keine chemische Umwandlung erfährt, sondern dass nur das überschüssige Wasser ausgetrieben wird. Daher wird der Leim auch nicht so hart wie die Duroplaste und kann auch nach dem Erkalten durch nachträgliches Erwärmen wieder erweicht werden. PVAc-Leime werden dort eingesetzt, wo keine außergewöhnlichen Ansprüche an die Festigkeit der Verbindung gestellt werden.

Weiterhin werden auch Leime auf Polyurethanbasis (PUR- Klebstoffe) eingesetzt. Bei diesen Klebstoffen erfolgt die Aushärtung durch Polyaddition. Der Einsatz in Hochfrequenzpressen ist bei diesem Klebstofftyp sehr wenig verbreitet.

Bei der Herstellung von Brettschichtholz unter Verwendung der Hochfrequenztechnik kann es wie bei jedem Verklebungsprozess zu Fehlverklebungen kommen. Werden diese Fehlverklebungen erst am Ende eines Fertigungsprozesses erkannt, sind in der Regel bereits etliche Meter unverkäuflicher Ware unter Erzeugung erheblicher Kosten produziert. Schlimmer noch ist es, wenn bei einer regelmäßig stattfindenden Prüfung eine eventuell schlecht verklebte Brettschichtholzcharge nicht erkannt wird und als Folge davon großer Schaden, möglicherweise sogar Personenschäden entstehen. Derartige Fehler können beispielsweise auf Verklebungsfehler zurückgeführt werden, bei denen z.B. die Wartezeit des Klebstoffes überschritten wurde oder die nötigen Aushärteparameter wie z.B. Leimfugentemperaturen nicht gegeben waren.

Derzeit wird die Leimfugentemperatur bei Brettschichtholz-Herstellerbetrieben durch die Bohrmethode überprüft. Hierbei wird ein Loch in die Leimfuge gebohrt und mittels eines Thermometers die Temperatur in dem Bohrloch gemessen. Aufgrund der mannigfaltigen Einflussfaktoren, wie zum Beispiel der Durchmesser des Bohrers, dessen Drehzahl, die Dauer des Bohrvorganges, die Bohrtiefe, die Genauigkeit der Bohrrichtung, der Ort des Bohrens, die die Bohrung durchführende Person, das verwendete Thermometer und dergleichen mehr, sind die erhaltenen Ergebnisse stark schwankend und beeinflussbar.

DE 696 05 801 T2 bezieht sich auf die Vorhersage von Eigenschaften einer Platte unter Verwendung eines spektroskopischen Verfahrens in Kombination mit einer multivariablen Eichung. Zunächst wird ein Rohholzmaterial oder eine Holzplatte selbst mit einem Feuchtigkeitsgehalt unter 10 % durch ein Spektraldaten lieferndes spektrometrisches Verfahren analysiert. Es erfolgt ein Vergleich der Spektraldaten mit Referenzspektraldaten, die durch das spektrometrische Verfahren von Referenzrohholzmaterial oder Referenzholzplatten mit einem Feuchtigkeitsgehalt unter 10 % erhalten wurden, wobei der Referenzspektraldaten anhand bekannter Parameter von aus dem Referenzrohholzmaterial hergestellten Holzplatten oder anhand bekannter Parameter der Referenzholzplatte mittels mulitvarianter Analyse kalibriert wurden.

WO 2013/023987 A1 hat ein luftgestütztes kontaktloses Ultraschallmessverfahren zur nicht zerstörenden Werkstoffprüfung zum Gegenstand. Es werden Defekte in laminierten Strukturen bestimmt, die eine Weite W sowie eine Anzahl n von Lamellen aufweisen und entlang einer Vielzahl von Klebeebenen miteinander verbunden sind. Mindestens ein Transmitter, der sich in einer bestimmten Transmitterdistanz befindet, strahlt Ultraschallstrahlen an unterschiedlichen Position ein und mindestens ein Empfänger, der sich in einer Sensordistanz befindet, empfängt die reflektierten Ultraschallstrahlen an verschiedenen Stellen der Laminatstruktur. Mittels des vorgeschlagenen Verfahrens können die Position sowie die Geometrie, beispielsweise Laminierungsdefekte, bildhaft dargestellt werden und es besteht die Möglichkeit, die laminierte Struktur willkürlicher Höhe und Länge einer individuellen Überprüfung in spezifischen Klebeebenen zu unterziehen.

WO 92/01540 bezieht sich auf eine Vorrichtung, mit der Holzkomponenten gefertigt werden. Dazu wird ein Erwärmungsprozess über Mikrowellen eingesetzt. Aufgrund unterschiedlicher Temperatur- und Feuchtigkeitsprofile und ungleichmäßiger Mikrowelleneintragungsmuster in einer Pressvorrichtung, weisen Holzbauteile unebene Dichteprofile auf, welches zu einer reduzierten Festigkeit, insbesondere im feuchten Zusatand der Holzprodukte geführt hat. Bevor die Holzbauteile die Pressvorrichtung erreichen, werden Feuchtigkeits- und Temperaturprofile derart ermittelt, dass im späteren Bauteil verbesserte Dichteprofile erhalten werden. Feuchtigkeits- und/oder Temperaturniveaus in bestimmten Bereichen werden erhöht oder herabgesetzt, so dass sich gewünschte Profile einstellen. Die Profile können derart eingestellt werden, dass beispielsweise durch die Zuführung von Heißdampf oder durch eine Wasserbesprühung oder eine bestimmte Kühlluftzufuhr entsprechend gleichmäßige Profile nach Durchlauf der Pressvorrichtung erhalten lassen.

### Darstellung der Erfindung

Der vorliegenden Erfindung liegt die Aufgabe zugrunde, ein Verfahren zur Beeinflussung und Regelung eines Verleimungsprozesses anzugeben, bei dem aus einer unmittelbar am Ende des Verleimungsprozesses durchgeführten Qualitätskontrolle unter Minimierung eines Ausschusses Korrekturdaten zur Beeinflussung des Verleimungsprozesses selbst gewonnen werden. Erfindungsgemäß wird ein Verfahren zur Beeinflussung und Regelung eines Verleimungsprozesses mit nachfolgenden Verfahrensschritten vorgeschlagen:
a) Vorgabe von Leimgangdaten an einen Leimassistenten (Software, die u.a. dazu dient, die Verarbeitungs- bzw. Produktionsparameter festzulegen) oder eine Steuereinrichtung,
b) Übermittlung ermittelter Parameter für eine Beleimung an eine Auftragsmaschine,
c) Vornahme einer Beleimung mittels eines Härterauftrags und/oder eines Leimauftrages von welchem eine oder beide Komponenten eine elektrische Leitfähigkeit aufweisen, auf Lamellen mindestens eines Brettschichtholzbauteils,
d) Taktweise oder kontinuierliche Verpressung mindestens eines gemäß Verfahrensschritt c) behandelten Brettschichtholzes in einer Pressvorrichtung,
e) Thermographie-Bild-Erfassung von Einzelfugen durch eine Thermographie-Kamera und Auswertung daraus bestimmter Fugentemperatur-Diagramme und
f) Detektion fehlerhafter Fugen innerhalb des Fugentemperatur-Diagrammes und Übermittlung von Korrekturdaten an den Leimassistenten oder die Steuerungsvorrichtung, und/oder die Auftragsanlage und/oder die Pressvorrichtung und/oder den Bediener zur Beeinflussung der Beleimungs- und/oder Prozessparameter, dadurch gekennzeichnet, dass lokal eine Dosierung der elektrisch leitfähigen Komponente, Leim und/oder Härter zur Energiezufuhr dort erhöht wird, wo eine zu niedrige Fugentemperatur gemäß des Fugentemperaturdiagrammes ermittelt wurde.

Durch das erfindungsgemäß vorgeschlagene Verfahren besteht die Möglichkeit der Optimierung eines Verleimungsprozesses dahingehend, dass innerhalb einer thermographischen Messung ermittelte Daten hinsichtlich erzeugter zu kalter oder unterbrochener Leimfugen unmittelbar in den Herstellungsprozess zurückgemeldet werden können. Die im Rahmen der aus der thermographischen Messung erzeugten Fugentemperatur-Diagramme ermittelbaren Korrekturdaten können sowohl an die Auftragseinrichtung als auch an die Pressvorrichtung, die innerhalb des Verleimungsprozesses durchlaufen werden, zurückgemeldet werden, so dass insbesondere eine leitfähige Komponente hinsichtlich ihrer Auftragsmenge oder der Veränderung ihrer Auftragsmenge an unterschiedlichen Stellen im Bauteil beeinflusst und korrigiert werden kann. Indirekt kann die Menge der betreffenden, leitfähigen Komponente auch durch das Mischungsverhältnis (meist Leim zu Härter) eingestellt werden. Damit kann Ausschuss verhindert sowie der der thermographischen Messung vorgeschaltete Verleimungsprozess weiter optimiert werden. Insbesondere können sich im Herstellungsprozess ergebende Schwachstellen, d.h. zu kalte Einzelfugen oder unterbrochene Einzelfugen durch die thermographische Messung sofort detektiert werden und entsprechende Schritte zur Nachprüfung und Fehlerbehebung eingeleitet werden. Die leitfähige Komponente kann gemäss der Erfindung je nach Beschaffenheit sowohl der Leim als auch der Härter sein.

In vorteilhafter Weiterbildung des der Erfindung zugrunde liegenden Gedankens wird gemäß Verfahrensschritt f) eine Durchschnittsfugentemperatur über jede Fuge des betreffenden Leimgangs innerhalb eines Infrarot-Scans, aufgenommen durch die Thermographie-Kamera, ermittelt. Aus dem Fugentemperatur-Diagramm, welches aus dem von der Thermographie-Kamera aufgenommenen Infrarot-Scan ermittelt wird, gehen diejenigen der Einzelfugen hervor, deren Durchschnittstemperatur unterhalb der empfohlenen durchschnittlichen Fugentemperatur liegt. Diese werden als Fehlfugen kenntlich gemacht und lokalisiert. Sofern die Temperatur entlang einer Einzelfuge zu stark schwankt, was über eine zulässige Standardabweichung eingegrenzt wird, wird dies als Fehler kenntlich gemacht.

Darüber hinaus leistet das erfindungsgemäß vorgeschlagene Verfahren eine weitere Verbesserung des Herstellungsprozesses. Aus dem aus dem Infrarot-Scan der Einzelfugen hervorgehenden Fugentemperatur-Diagramm werden Einzelfugen detektiert, die entlang ihres Fugenverlaufes in Durchlaufrichtung sowohl durch die Auftragsvorrichtung als auch durch die Pressvorrichtung verursachte Unterbrechungen aufweisen. Derartige, Unterbrechungen aufweisende Einzelfugen werden als Fehlfugen kenntlich gemacht und lokalisiert. Weiterhin wird an der erfahrungsgemäß kältesten Stelle in einer Leimfuge, abhängig von der Bauart der Presse und weiterer konstruktiver Gegebenheiten, von der Oberflächentemperatur auf die bei einer manuellen Temperaturkontrolle zu erwartende Kerntemperatur zurückgerechnet. Die Fuge erwärmt sich in der Lamellenmitte (bezogen auf die Lamellenbreite) schneller und stärker als im Randbereich. Somit kann durch einen Algorithmus von der Oberflächentemperatur auf die Kerntemperatur geschlossen werden.

Korrekturdaten, die Informationen über unterbrochene Einzelfugen oder über Einzelfugen enthalten, deren Temperatur einer Mindestdurchschnittstemperatur nicht entspricht, d.h. diese unterschreitet, und die demzufolge als potentielle Schwachstellen anzusehen sind, werden in den Herstellungsprozess zurückgekoppelt um den Fehler bei folgenden Verpressungen zu verhindern. Eine Rückkopplung kann zum einen an den Leimassistenten oder z.B. eine Steuervorrichtung erfolgen. Dieser ermöglicht es, die Auftragsanlage dementsprechend zu steuern, wodurch der Auftrag der Komponenten beeinflusst wird. Da die Fugentemperaturen wie auch die Lage der fehlerhaften Fugen im Bauteil bekannt sind, kann somit gezielt Einfluss auf den Auftrag der einzelnen Komponenten genommen werden, so dass die Korrekturdaten, die aus der Auswertung des Fugentemperatur-Diagramms erhalten werden, direkt in den Herstellungsprozess einfließen können.

Die Auftragsmenge der leitfähigen Komponente kann durch die Leimflottenauftragsmenge und/oder durch das Mischungsverhältnis der Komponenten lokal in Bezug auf bestimmte Stellen in einer Pressenfüllung, sei es im Filmauftrag, sei es im Schnurauftrag, verändert werden.

Durch gezielte Beeinflussung eines Leimflottenauftrages gemäß den über die Thermographie-Kamera erhaltenen Fugentemperatur-Diagrammen lässt sich der Herstellungsprozess beeinflussen. Durch die höhere Dosierung der leitfähigen Komponente kann gemäß der Erfindung, gezielt eine Erhöhung der Temperatur in den gewünschten Leimfugen erfolgen. Da aus den Fugentemperatur-Diagrammen auch der Ort der "kalten" Einzelfuge bekannt ist, kann gezielt Einfluss auf die Optimierung des Leimflottenauftrages, insbesondere einer leitfähigen Komponente, an einer bestimmten Stelle genommen werden. Durch das erfindungsgemäß vorgeschlagene Verfahren kann eine Beleimung durch eine Kombination von Härterautrag und Leimauftrag in verschiedenen Variationsmöglichkeiten erfolgen: So besteht die Möglichkeit, die Komponenten Leim und Härter als Schnur- oder Filmauftrag zu realisieren, wobei jegliche Kombination dieser Auftragsformen denkbar ist. Z.B Härter als Schnur und Leim als Film.

Zu den Parametern hinsichtlich des Verleimungsprozesses, sei es in einer getakteten Presseinrichtung, sei es in einer Durchlaufpresseinrichtung, gehen mindestens folgende Einstellungen in Bezug auf hochfrequente Presseinrichtungen ein: Eine minimale Lamellenlänge (Iₘᵢₙ), eine maximale Lamellenlänge (Iₘₐₓ), eine minimale Lamellentemperatur (Tₘᵢₙ), eine maximale Lamellentemperatur (Tₘₐₓ) sowie Safe-Bereich-Grenzen (Breite und Stärke)

Innerhalb einer Takt-Version können in Abhängigkeit von der Lamellenlänge vordefinierte Mengeneinstellungen vorgenommen werden, d.h. eine längste Lamelle bestimmt eine Anzahl von Presstakten in der Pressvorrichtung. In Abhängigkeit von der Lamellentemperatur sollen vordefinierte Härtereinstellungen vorgenommen werden. Bei der Durchlaufversion verhält es sich ähnlich. Hier definiert die Vorschubgeschwindigkeit des Pressgutes, mit welchem dieses die Presse durchläuft, in Kombination mit der Länge der längsten Lamelle die Verweildauer in der Presse und somit im hochfrequenten Feld. Die vordefinierten Mengen- und Härtereinstellungen können bei beiden Versionen der Pressvorrichtung jeglichen Funktionen entsprechen, so z.B. linear, blockweise oder auch polynomisch. Auch ist es denkbar, dass sich die Mengen- und Härtereinstellungen automatisch durch die zugrunde liegenden Korrekturdaten der vergangenen Pressvorgänge, mit dem Ziel einer homogenen Temperaturverteilung sämtlicher Einzelfugen anpassen. Das Vordefinieren der Mengen- und Härtereinstellungen würde somit entfallen, da sich diese prozessoptimiert generieren.

### Vorteile der Erfindung

Durch das erfindungsgemäß vorgeschlagene Verfahren besteht die Möglichkeit, im Rahmen von thermographischen Messungen zugängliche Informationen so aufzubereiten, dass diese Informationen als Korrekturdaten in den Herstellungsprozess, d.h. einen Verleimungsprozess zur Herstellung von Brettschichtholz, rückgekoppelt werden können. Aus den thermographischen Messungen gehen Daten hinsichtlich von Fehlfugen, deren Lage und deren Position hervor, so dass gezielte Rückmeldungen sowohl in den Leimassistenten bzw. dessen Steuereinheit, die Auftragsmaschine, den Bediener sowie in die Pressvorrichtung zur Herstellung des Brettschichtholzes rückgemeldet werden können. Dadurch kann unmittelbar auf sich ergebende Schwachstellen im Herstellungsprozess reagiert werden, so dass die Produktionssicherheit erhöht wird.

Die aus der thermographischen Messung hervorgehenden Korrekturdaten können somit bereits in einer "Closed-Loop"-Messung in den Herstellprozess zurückgegeben werden und dazu eingesetzt werden, gezielt sich abzeichnende Schwachstellen auszumerzen.

Das erfindungsgemäß vorgeschlagene Verfahren bietet neben der bereits erwähnten Reduzierung möglichen Ausschusses weitere immense Vorteile bezüglich der Qualitätssicherung. Da jeder Pressvorgang erfasst wird und die Thermographiebilder automatisiert aufgenommen und ausgewertet werden, kann eine lückenlose Dokumentation zur Verfügung gestellt werden, mit der Besonderheit, dass die Fugentemperatur in jeder Stelle sämtlicher einzelner Leimfugen analysiert werden kann. Durch die gezielte Einflussnahme an Schwachstellen, d.h. Stellen, die aus konstruktiven oder anderen Gründen eine geringere Erwärmung der Leimfugen aufweisen, können diese nunmehr erheblich sicherer gestaltet werden. Hiermit kann das Potenzial von Produktionen gezielter ausgeschöpft werden, ohne Einbußen bei der Sicherheit in Kauf nehmen zu müssen. Potenziale können zum Beispiel die Optimierung der Leim- und Härterauftragsmenge, des Mischungsverhältnisses dieser Komponenten, der Wartezeit oder der Presszeit sowie die damit zusammenhängende Energieeinsparung sein. Alle diese Punkte gehen in eine ökonomische Optimierung eines Produktionsprozesses ein.

### Kurze Beschreibung der Zeichnungen

Anhand der Zeichnungen wird die Erfindung nachstehend eingehender beschrieben:
Es zeigt:
- Figur 1: einen schematisch wiedergegebenen Ablauf eines Verleimungsprozesses und die im Wesentlichen an diesem beteiligten Komponenten,
- Figur 2: eine Messanordnung mit einer Thermographie-Kamera, die in Durchlaufrichtung einer Pressenfüllung, am Beispiel einer Taktpresse, gesehen hinter einer Pressvorrichtung angeordnet ist,
- Figuren 3, 4, und 5: Details einer Auftragseinrichtung für kombinierten Härter- und Leimauftrag,
- Figur 6.1+6.2: beispielhafte Mengen- und Härtervariationen an einer Durchlaufpresse und an einer Taktpresse
- Figur 7: einen durch eine Thermographie-Kamera aufgenommenen Infrarot-Scan von Einzelfugen,
- Figur 8: ein zu Figur 7 gehörendes Fugentemperatur-Diagramm mit Gutfugen,
- Figur 9: ein Infrarot-Scan einer Thermographie-Kamera mit Einzelfugen,
- Figur 10: ein zu Figur 9 gehörendes Fugentemperatur-Diagramm mit Durchschnittfugentemperaturen, Gutfugen und fehlerhafter Fugen aufgrund zu niedriger Fugentemperatur (schraffierte Balken im Diagramm),
- Figur 11: einen Infrarot-Scan von Einzelfugen, bei denen fehlerhafte Fugen Unterbrechungen aufweisen und
- Figur 12: ein zu Figur 11 gehörendes Fugentemperatur-Diagramm mit Gutfugen, welche trotz Unterbrechungen in Fugen (vgl. Figur 11) ausreichende Durchschnittstemperaturen aufweisen.

### Ausführungsvarianten

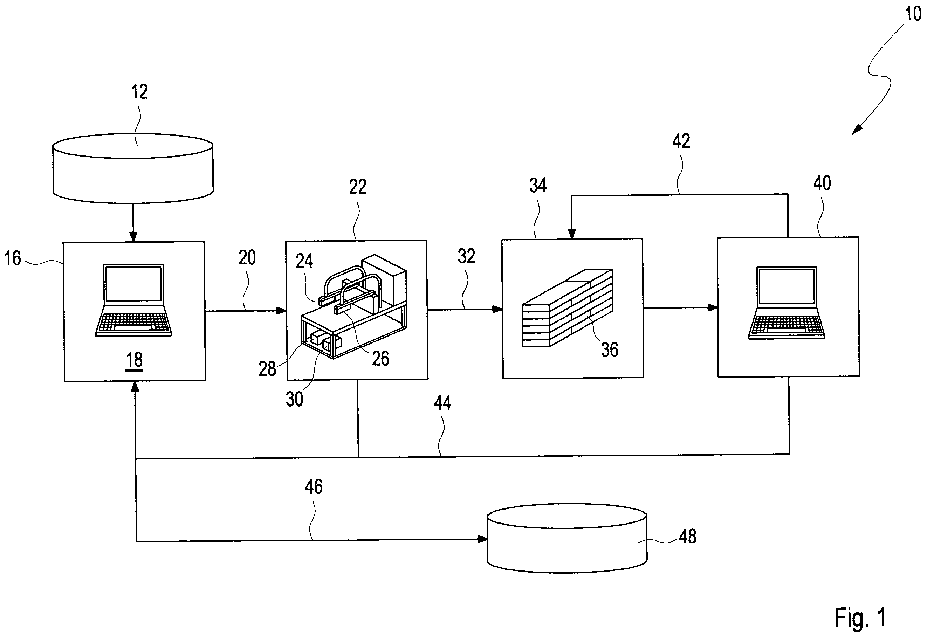

Der Darstellung gemäß Figur 1 ist schematisch ein Herstellprozess eines Brettschichtholzes zu entnehmen sowie die wesentlichen an diesen beteiligten Komponenten.

Innerhalb eines Herstellprozesses 10 zur Erzeugung von Brettschichtholz 36 werden Leimgangdaten 12 an einen Leimassistenten 16 übermittelt. Unter Leimassistent ist im vorliegenden Zusammenhang eine Eingabeeinheit, beispielsweise ein PC zu verstehen, an dem eine Vorgabe bzw. Datenaufbereitung 18 erfolgt. Die abhängig von den Leimgangdaten 12 aufbereiteten Daten werden im Rahmen einer Parameterübermittlung 20 an eine Auftragsanlage 22 übermittelt. Leimgangdaten 12 sind im vorliegenden als Herstell- bzw. Produktionsdaten zu verstehen, die von Bedeutung sind, um den Beleim- und den Pressvorgang erfolgreich zu planen und letztendlich durchzuführen. So zum Beispiel die Lamellenanzahl, die Bauteilanzahl in einer Pressenfüllung, die vorliegenden Lamellentemperaturen, die Vorschubgeschwindigkeit beim Beleimen, die minimale und maximale Lamellenlänge, die Stärke und Breite der Lamellen, die zu befüllende Presse sowie weitere Parameter.

Parameterübermittlung 20 übermittelt die aus den Leimgangdaten ermittelten Parameter, welche weiterhin benötigt werden, um die Beleimung und Verpressung durchzuführen, wie z.B. Leimflottenauftragsmenge, Mischungsverhältnis der Komponenten und somit den Anteil der leitfähigen Komponente, bezogen auf jede Leimfuge.

Bei der Auftragsanlage 22, die innerhalb des Herstellprozesses 10 gemäß der Darstellung in Figur 1 nur schematisch wiedergegeben ist, erfolgt ein Auftrag 26 eines Härters sowie ein Leimauftrag 24 auf die Oberseite einer entsprechend präparierten, insbesondere gehobelten Holzkomponente. Bei der Auftragsanlage 22 kann es sich um eine Einheit, eine beispielsweise auf Rollen verfahrbare Einheit gemäß der schematischen Wiedergabe in Figur 1, handeln. Diese umfasst zur Förderung des Härters im Rahmen des Auftrags 26 sowie zur Förderung des Leimes in Rahmen eines Leimauftrages 24 jeweils eine Förderpumpe 28, 30. Die beiden Förderpumpen 28, 30 sind Bestandteile der Auftragsanlage 22.

Nach Auftrag des Härters im Rahmen des Auftrages 26 und nach dem Auftrag des Leimes 24 erfolgt eine Verpressung. Dazu werden die miteinander zu verpressenden einzelnen Komponenten in einer Pressvorrichtung 34, bei der es sich insbesondere um eine Hochfrequenzpressvorrichtung handelt, miteinander verpresst. Erhalten wird ein in Figur 1 durch Bezugszeichen 36 bezeichnetes Brettschichtholz 36. Dieses besteht aus mehreren Lagen, wobei die einzelnen Lagen aus unterschiedlichen, durch eine Keilzinkung verbundenen Einzelbrettern bestehen, die entlang von Einzelfugen stoffschlüssig miteinander verbunden sind.

Nach einer Verpressung wird das erhaltene verpresste Brettschichtholz 36 über eine in Figur 1 nicht dargestellte Thermographie-Kamera erfasst. Es erfolgt eine Thermographie-Bild-Erfassung 40 sowie eine Auswertung der von der Thermographie-Kamera aufgenommenen Fugenbilder von Einzelfugen des in der Pressvorrichtung 34 erzeugten Brettschichtholzes 36. Die Verpressung innerhalb der Pressvorrichtung 34, bei der es sich um eine Durchlaufpresse oder um eine Taktpresse handeln kann, wird unter Einwirkung des hochfrequenten Feldes der erforderliche Pressdruck über die erforderliche Presszeit aufrechterhalten. Abhängig von der Bauteilbreite, der Anzahl der Leimfugen und der Länge der zu verpressenden Bauteile liegt die Presszeit bzw. Verweildauer im hochfrequenten Feld bei erforderlichem Pressruck etwa zwischen einer und vier Minuten.

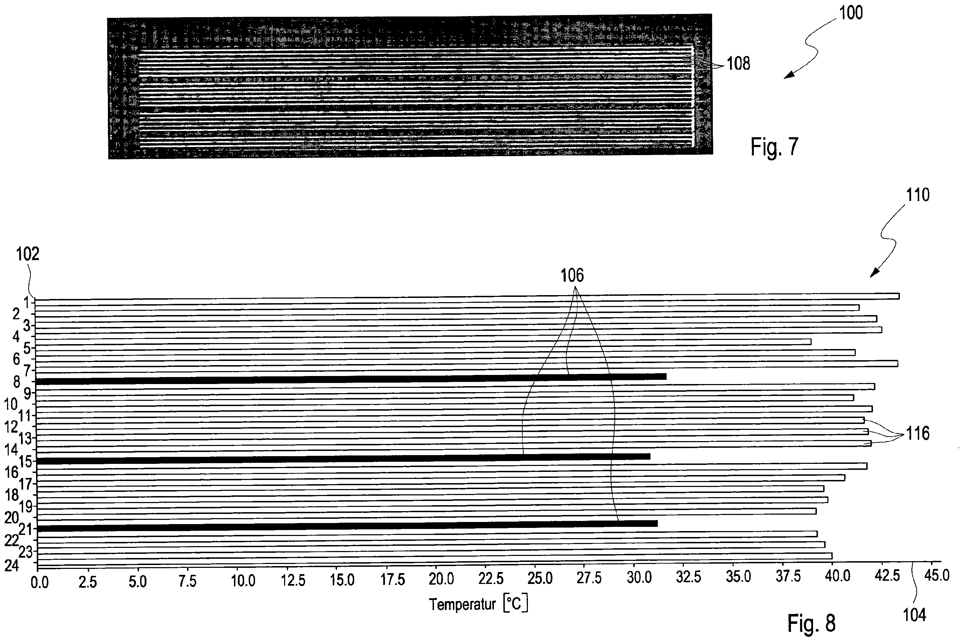

Im Rahmen der Thermographie-Bild-Erfassung und Auswertung 40 werden aus Thermographie-Bildern, aufgenommen mit der Thermographie-Kamera 38, Fugentemperatur-Diagramme 110, vgl. Darstellungen gemäß der Figuren 7 bis 12, erzeugt. Aus den Fugentemperatur-Diagrammen 110 gehen Daten hervor, die im Rahmen einer Datenrückführung 42 entweder an die Pressvorichtung 34, bei der es sich bevorzugt um eine Hochfrequenzpressvorrichtung 34 handelt, oder im Rahmen einer Datenrückführung 44 an den Leimassistenten 16 bzw. eine Steuerungsvorrichtung 16 zur Optimierung der Datenvorgabe 18 innerhalb des Leimassistenten zurückgeführt werden. Darüber hinaus kann die Datenrückführung 44 parallel oder auch gezielt lediglich direkt an die Auftragsanlage 22 und dort insbesondere an die Steuerungskomponenten zur Steuerung des Auftrages der leitfähigen Komponente, beispielsweise eines Härters, rückgekoppelt werden. Die Dosierung der leitfähigen Komponente stellt ein entscheidendes Kriterium für die Abbindetemperaturen der Kombination aus Leim und Härter in den Einzelfugen des Brettschichtholzes 36 dar und durch entsprechende Dosierung kann die Abbindetemperatur des Gemisches aus Leim und Härter gezielt beeinflusst werden. Insbesondere können durch die Datenrückführung 42, 44 gezielt Änderungen im Herstellprozess 10 unmittelbar bei Prozessablauf erreicht werden, so dass im Rahmen der Thermographie-Bild-Erfassung und Auswertung 40 detektierte Fehlstellen durch eine entsprechende Korrektur der Parameterübermittlung 20 bei nachfolgend zu fertigenden Bauteilen vermieden werden können.

Der Vollständigkeit halber sei erwähnt, dass die über die Thermographie-Bild-Erfassung-und-Auswerteeinheit 40 erhaltenen Korrekturdaten, die im Rahmen der Datenrückführung 42, 44 in den Herstellungsprozess 10, d.h. an die Komponenten rückgekoppelt werden im Rahmen einer Dokumentation 46 in eine Datenbank 48 (Leimbuch) übermittelt und dort dauerhaft gespeichert werden, so dass diese zur Dokumentation des Herstellprozesses 10 vorliegen und auf diese Dokumentation 46 zurückgegriffen werden kann. Aus der Datenbank 48 kann das Leimbuch erstellt werden, welches alle dokumentierungspflichtigen Daten zum Herstellvorgang beinhaltet.

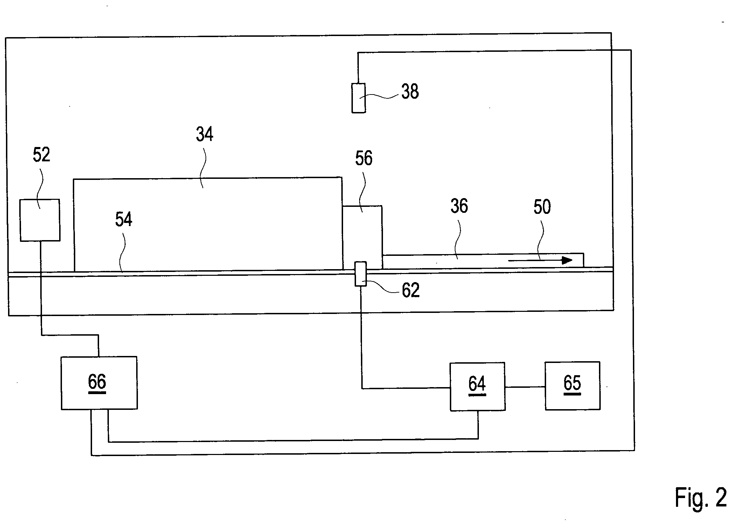

Figur 2 zeigt die Anordnung einer Thermographie-Kamera, die zur Thermographie-Bild-Erfassung dient.

Aus der Darstellung gemäß Figur 2 geht hervor, dass eine Thermographie-Kamera 38 einer Pressvorrichtung 34, bei der es sich vorzugsweise um eine Hochfrequenzpresseinrichtung handelt, zugeordnet ist. Die Thermographie-Kamera 38 ist in Bezug auf die Durchlaufrichtung 50 des verpressten Brettschichtholzes 36 nach der Pressvorrichtung 34 angeordnet. Aus der Darstellung gemäß Figur 2 geht hervor, dass sich die Thermographie-Kamera 38 oberhalb einer Haube 56, die der Abschirmung des hochfrequenten Feldes dient, befindet. Mit Bezugszeichen 52 ist ein mit einer Rechnereinheit 66 kommunizierender Monitor 52 bezeichnet. Die Rechnereinheit 66 steht darüber hinaus mit einer Steuerung 64 in Verbindung. Bei der Steuerung 64 handelt es sich beispielsweise um eine speicherprogrammierbare Steuerung (SPS) oder Ähnliches.

Figur 2 ist des Weiteren zu entnehmen, dass die Steuerung 64 mit einer Lichtschranke 62 verbunden ist, die detektiert, ob sich ein Brettschichtholzbauteil 36 unter der Thermographie-Kamera 38 befindet oder nicht.

Aus der Darstellung gemäß Figur 2 geht hervor, dass sich das Brettschichtholz 36 in Durchlaufrichtung 50 auf der Förderebene 54 durch die bevorzugt als Hochfrequenzpressvorrichtung ausgeführte Pressvorrichtung 34 befördert und dabei den Erfassungsbereich der Thermographie-Kamera 38 passiert.
Die Steuerung 65 kommuniziert die Zustände Presse offen, Presse geschlossen, Vorschub an/aus. Des Weiteren wird die Identifikationsnummer des Pressvorgangs übergeben.

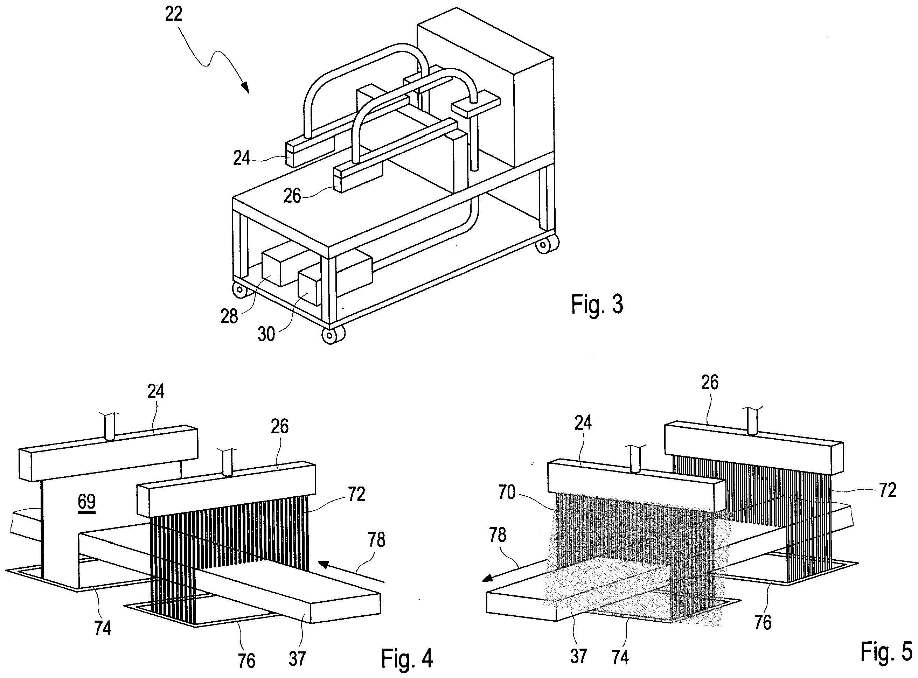

Den Darstellungen gemäß der Figuren 3, 4, 5 sind verschiedene Ansichten einer Auftragsanlage zu entnehmen, über die separat voneinander ein Härterauftrag 26 sowie ein Leimauftrag 24 auf eine Lamelle 37.

Der Darstellung gemäß Figur 3 ist eine Auftragsanlage 22 zu entnehmen. Die Auftragsanlage 22 umfasst einen Auftragskopf zur Durchführung eines Härterauftrags 26, ferner umfasst die Auftragsanlage 22 einen Leimkopf zur Durchführung eines Leimauftrages 24. Der Härterauftrag 26 erfolgt mittels einer ersten Förderpumpe 30, die den Härter aus einem in Figur 3 nicht dargestellten Vorratsbehälter zum Härter Auftrag führt. Analog wird Leim über eine zweite Förderpumpe 28 zum Leimauftrag 24 gefördert. Beim Durchlauf der zu beleimenden Lamelle erfolgt der Härterauftrag 26 vor dem Leimauftrag 24.

Der Darstellung gemäß Figur 4 ist zu entnehmen, dass über die Auftragsanlage 22 gemäß Figur 3 im in Figur 4 dargestellten Betriebsmodus der Härterauftrag 26 im Schnurauftrag 72 erfolgt, während gemäß der Darstellung in Figur 4 der Leimauftrag 24 in Gestalt eines Filmauftrages 69 erfolgt. Der Filmauftrag 69 und der Schnurauftrag 72 erfolgen sequentiell nacheinander auf die Oberseite von zu einem Brettschichtholz 36 zu verleimenden Lamellen 37. Deren Oberseite ist zur Begünstigung der Herstellung der Verleimung speziell behandelt, insbesondere gehobelt.

Figur 5 zeigt eine Auftragsanlage, bei der der Härterauftrag 26 in Form eines Schnurauftrags 72 erfolgt, während der Leimauftrag 24 analog zur Darstellung gemäß Figur 4 als Schnurauftrag 70 erfolgt.

Sowohl dem Härterauftrag 26 als auch dem Leimauftrag 24 sind Auffangwannen 74, 76 zugeordnet, über welche nicht benutzter, verbleibender, überschüssiger Härter bzw. verbliebener überschüssiger Leim aufgefangen werden und erneut im Rahmen einer Rezirkulation über den Härterauftrag 26 bzw. den Leimauftrag 24 verarbeitet werden können. Aus den Darstellungen gemäß der Figuren 4 und 5 ergibt sich, dass durch die gewählte Breite der Auftragsköpfe für den Härterauftrag 26 und den Leimauftrag 24 auch breitere als in den Figuren 4 und 5 dargestellte Lamellen 37 über deren gesamte Breite entweder im Filmauftrags- oder im Schnurauftragsmodus benetzt werden können.

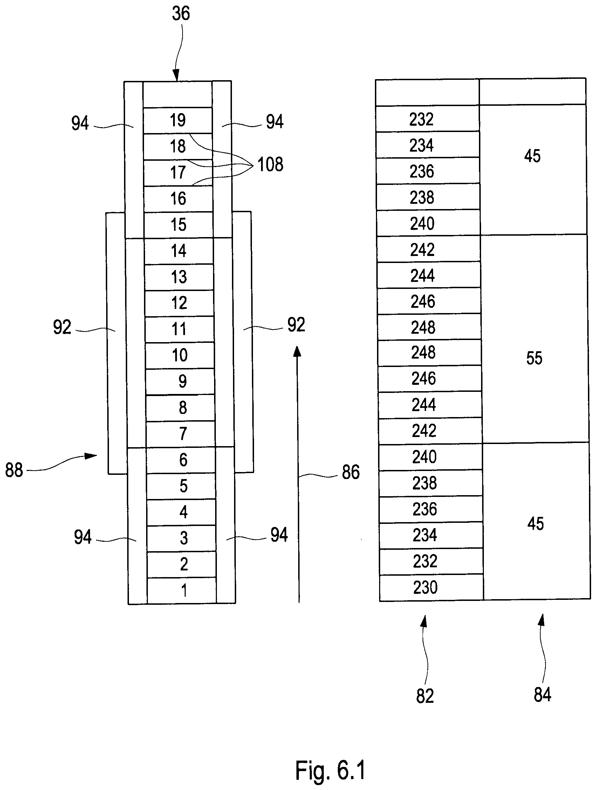

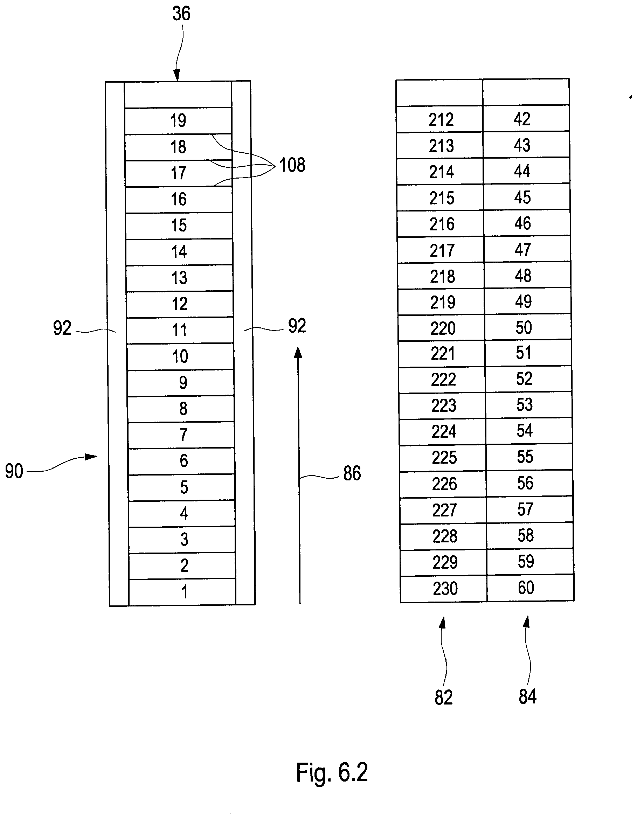

Die Figuren 6.1 und 6.2 zeigen beispielhafte Leimflottenauftragsmengen 82 und Härterregulierungen 84 am Beispiel einer Pressenfüllung 36, die in einer Durchlaufpresse 88 verarbeitet wird und einer Pressenfüllung 36, die in einer Taktpresse 90 erzeugt wird.

Der Darstellung gemäß Figur 6.1 ist zu entnehmen, dass im Falle einer als Durchlaufpresse 88 beschaffenen Pressvorrichtung 34 ein oder mehrere Brettschichtholzbauteile 36 (Pressenfüllung) erzeugt werden können, die eine Anzahl von Lamellen umfassen. Entsprechend der Anzahl von Lamellen der Brettschichtholzbauteile 36, vergleiche Fugenanzahl 86, umfassen diese gemäß der Darstellung in Figur 6.1 19 Einzelfugen 108. Die Durchlaufpresse 88 umfasst Kondensatorplatten 92, die stationär angeordnet sind, sowie Kondensatorplatten 94, die sich variabel an die Höhe der zu erzeugenden Brettschichtholzbauteile 36 anpassen lassen. Wie aus einem Leimauftrag 82 in g/m² hervorgeht, weisen die Einzelfugen 108, vgl. Position 1 bis 19, entsprechend der Fugenanzahl 86 unterschiedliche Leimflottenauftragsmengen 82 in g/m² auf, die von der Unterseite in die Mitte der Pressenfüllung, bzw. von der Oberseite in die Mitte allmählich zunehmen. Die maximale Leimflottenmenge in g/m² befindet sich in der Mitte der Pressenfüllung 36 im Bereich der Einzelfugen 108, Nr. 10 und 11. Demgegenüber ist eine leitfähige Komponente 84 - beispielsweise ein Härter - in Bezug auf die Einzelfugen 1 bis 6 mit 45 Gewichtsanteilen, in Bezug auf die Einzelfugen 7 bis 14 mit 55 Gewichtsanteilen und in Bezug auf die Einzelfugen 15 bis 19 mit 45 Gewichtsanteilen aufgebracht. Dies bedeutet, dass die Zugabe der leitfähigen Komponente im Inneren der Pressenfüllung 36 höher ist als in dessen Randbereichen auf der Ober- und der Unterseite.

Der Darstellung gemäß Figur 6.2 sind die Verhältnisse hinsichtlich einer Leimflottenmengen- und Härterregulierung 84 an einer Pressenfüllung 36 zu entnehmen, die an einer Taktpresse hergestellt wird.

Die in Figur 6.2 dargestellte Pressenfüllung 36 umfasst ebenfalls eine entsprechend der Fugenanzahl 86, Nr. 1 bis 19. Die Taktpresse 90 umfasst Kondensatorplatten 92, über welche ein hochfrequentes Feld erzeugt wird, welches auf die Einzelfugen 108 entsprechend ihrer Fugenanzahl 86 einwirkt. In der in Figur 6.2µ dargestellten Pressenfüllung 36 nimmt der Leimflottenauftrag 82, wie beispielhaft dargestellt, von der Einzelfuge Nr. 1 mit 230g/m² bis zur Einzelfuge Nr. 19 bis auf 212g/m² ab. Korrespondierend dazu verhält sich die Mengenzugabe der leitfähigen Komponente in Gestalt eines Härters in Gewichtsanteilen, die ausgehend von der Einzelfuge 108 mit Nr. 1 mit 60 Gewichtsteilen bis auf 42 Gewichtsteile an der Einzelfuge 108 mit Nr. 19 bezeichnet in Pressenfüllung 36 in vertikaler Richtung von unten nach oben abnimmt.

Figur 7 zeigt einen mittels einer Thermographie-Kamera aufgenommenen Infrarot-Scan von Einzelfugen entlang einer Pressenfüllung 36.

Der Darstellung gemäß Figur 7 ist ein Infrarot-Scan 100 zu entnehmen, der mittels der in Figur 2 oberhalb der Förderebene 54 angeordneten Thermographie-Kamera 38 erstellt wurde. Wie aus dem Infrarot-Scan 100 hervorgeht, umfasst dieser mehrere Einzelfugen 108. In der Thermographie-Bild-Erfassung-und-Auswerteeinheit 40, wie sie schematisch in Figur 1 angedeutet ist, wird ein Fugentemperatur-Diagramm 110 (vgl. Figur 8) erstellt. Im Fugentemperatur-Diagramm 110 gemäß der Darstellung in Figur 8 ist die Fugenanzahl 102, im vorliegenden Fall von Fuge 1 bis Fuge 24, über eine Fugentemperatur 104 aufgetragen. Die Länge der einzelnen den jeweiligen Fugen 1 bis 24 zugeordneten Balken entspricht der aus dem Infrarot-Scan 100 ermittelten Durchschnittstemperaturen in den Einzelfugen 108. Aus der Darstellung gemäß Figur 8, d.h. dem dort wiedergegebenen Fugentemperatur-Diagramm 110, geht hervor, dass die durchschnittliche Temperatur der Einzelfugen als ausreichend hoch angesehen wird, da diese in keiner Weise als fehlerhaft markiert werden. Die dunklen (hier schwarzen) Balken stellen die Trockenfugen 106 dar, welche die einzelnen Brettschichtholzbauteile (hier 4 Stück) in dieser Pressenfüllung voneinander trennt. Da hier kein Klebstoff aufgetragen wurde, erfolgte keine Erwärmung, was im Fugentemperatur-Diagramm 110 kenntlich gemacht, aber bewusst nicht als fehlerhafte Fuge dargestellt wird, weil durch die Leimgangdaten bekannt ist, dass es sich um Trockenfugen handelt. Die Temperaturen der Trockenfugen 8, 15 und 21, sind im Infrarot-Scan 100 gemäß Figur 7 aufgrund zu geringer Temperaturen dunkel geblieben. Die Oberflächentemperaturen liegen hier zwischen 30°C und 32,5°C, während die Temperaturen der Fugen 1 bis 7, 9 bis 14, 16 bis 20 sowie 22 bis 24 zwischen 39°C und 40°C liegen, d.h. eine für die Verleimung ausreichend hohe Temperatur aufweisen. Aus dem Fugentemperatur-Diagramm 110 gemäß der Darstellung in Figur 8 kann somit abgeleitet werden, dass bei den beleimten Einzelfugen 108 gemäß des Fugentemperatur-Diagrammes 110 ausreichende Temperatur für die Verleimung sichergestellt ist.

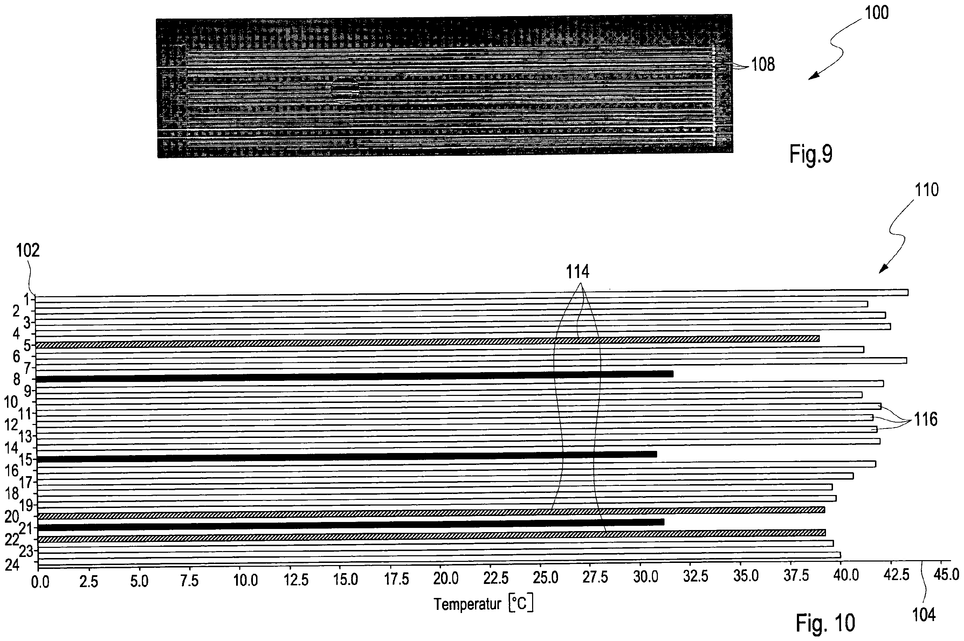

Figur 9 zeigt einen Infrarot-Scan 100, bei dessen Auswertung das in Figur 10 dargestellte Fugentemperatur-Diagramm erhalten wird.

Auch der Infrarot-Scan 100 gemäß der Darstellung in Figur 9 zeigt eine Anzahl von Einzelfugen 108. Es wird kenntlich gemacht, dass die Fugen 5, 20 und 22 fehlerhaft sind, was in Figur 10 schraffiert, als fehlerhafte Fuge 114 dargestellt ist. Die durchschnittliche Fugentemperatur der fehlerhaften Fugen liegt unterhalb der empfohlenen durchschnittlichen Fugentemperatur, welche in dem Programm vordefiniert werden kann. Die Auswertung des Infrarot-Scans 100 gemäß der Darstellung in Figur 9 führt zu einer Durchschnittsfugentemperatur der Einzelfugen 108 im Fugentemperatur-Diagramm 110 gemäß der Darstellung in Figur 10, wobei in diesem Falle eine Durchschnittsfugentemperatur aller "Gutfugen" 116 zwischen 37°C und 40,0°C liegt. Die Durchschnittsfugentemperatur ist in Bezug auf die Einzelfugen 5, 20 und 22 im Fugentemperatur-Diagramm 110 schraffiert, also fehlerhaft dargestellt und liegt unter 39°C, was in diesem Fall der vordefinierte Grenzwert ist. Die Einzelfugen 8, 15 und 21 stellen Trockenfugen 106 dar, in denen keine Erwärmung stattfinden konnte, da kein Leim und kein Härter appliziert wurden. Das Fugentemperaturdiagramm 110 gemäß der Darstellung in Figur 10 dient als Warnhinweis darauf, dass in den Einzelfugen 108 mit den Nr. 5, 20 und 21 die vorgegebenen Durchschnittsfugentemperatur nicht erreicht wurde. Eine Prüfung der Bauteile ist nötig.

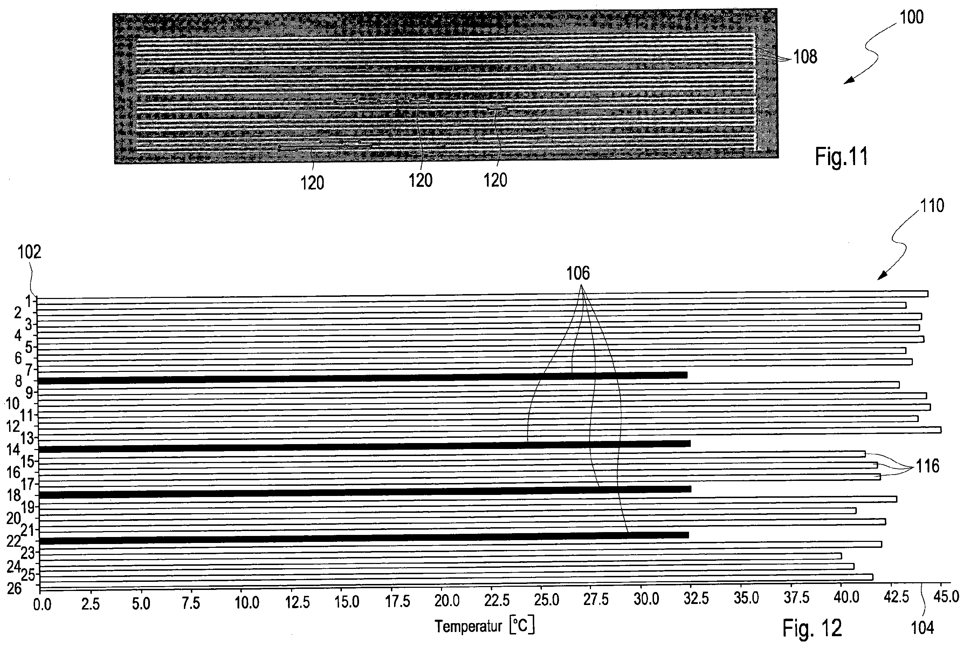

Figur 11 zeigt einen Infrarot-Scan 100 von Einzelfugen zwischen Lamellen 37 mehrerer Brettschichtholzbauteile 36 und Figur 12 ein auf den Infrarot-Scan 100 gemäß der Darstellung in Figur 11 zurückgehendes Fugentemperatur-Diagramm.

Aus der Darstellung in Figur 11 geht hervor, dass die Einzelfugen 108 fehlerhafte Fugen 114 umfassen, entlang deren Verlauf Unterbrechungen 120 vorliegen. Da Unterbrechungen 120 in Darstellung 100 eine fehlerhafte Verleimung zur Folge haben, weil an den betroffenen Stellen keine Erwärmung der Leimfuge stattgefunden hat, wird dies in Darstellung 100 als Fehler kenntlich gemacht. Eine jede im Infrarotscan 100 gemäß der Darstellung in Figur 11 detektierte fehlerhafte Fuge 114 weist mindestens eine Unterbrechung 120 auf. Figur 12 zeigt keine fehlerhaften Fugen, da hier nur die durchschnittlichen Fugentemperaturen ausgewertet werden, welche trotz der Unterbrechungen 120 in den Fugen 15, 17, 24, 25 und 26 ausreichend sind. Die Fugen 8, 14, 18 und 22 weisen erneut keine Erwärmung auf, da es sich hier um Trockenfugen 106, also nicht beleimte Fugen handelt, was keinen Fehler wiederspiegelt.

Ausgehend von dem Infrarot-Scan 100 gemäß der Darstellung in Figur 11 wird das in Figur 12 dargestellte Fugentemperatur-Diagramm 110 ermittelt. In diesem sind in Balkenform die Fugentemperaturen 104 über die Fugenanzahl 102 aufgetragen. Es ergibt sich, dass in den Einzelfugen 8, 14, 18 und 22 jeweils die in den Figuren 11 kenntlich gemachten Unterbrechungen 120 vorliegen. Aus diesem Grunde sind diese Einzelfugen 8, 14, 18 und 22 als Fehlfugen 114 gekennzeichnet, während in den im Fugentemperatur-Diagramm 110 gemäß Figur 11 dargestellten Gutfugen 116 gemäß des Infrarot-Scans 100 gemäß der Darstellung in Figur 11 Temperaturen erreicht werden, die oberhalb der 40,0°C-Schwelle liegen. Dies bedeutet, dass in den Gutfugen 116 des Fugentemperatur-Diagrammes 110 ein korrekter Verleimungsverlauf sichergestellt ist, wohingegen in den fehlerhaften Fugen 114 Unterbrechungen 120 vorliegen.

Die aus den Fugentemperatur-Diagrammen gemäß den Figuren 8, 10 und 12 hervorgehenden Informationen beruhen auf den jeweils zugehörigen Infrarot-Scans 100 gemäß der Figuren 7, 9 und 11. Die Informationen über die Einzelfugen 108, in denen Unterbrechungen 120 vorliegen oder in denen eine empfohlene Durchschnittsfugentemperatur nicht erreicht wird, können im Rahmen einer Datenrückführung 42, 44 (vgl. Darstellung gemäß der Figur 1) entweder an den Leimassistenten 16 bzw. die zugehörigen Steuereinheit 64 und unmittelbar an die Auftragsanlage 22, insbesondere dort dem Auftrag der leitfähigen Komponente und/oder der Pressvorrichtung 34, die bevorzugt als Hochfrequenzpresseinrichtung ausgebildet ist, zugeführt werden. Da aus den Fugentemperatur-Diagrammen 110 gemäß der Figuren 8, 10 und 12 bekannt ist, in welchen der Einzelfugen 108 Fehlstellen 120 vorliegen, bzw. eine korrekte Verleimung nicht ermöglichende, zu geringe Fugentemperaturen vorliegen, kann entsprechend, beispielsweise am Härterauftrag 26, nachjustiert werden. Dies bedeutet, dass über den Auftrag der leitfähigen Komponente an der Auftragsanlage 22, beispielsweise an einer bestimmten Stelle über die Breite des Bauteils gesehen, einer Einzelfuge 108 gemäß der Fugenanzahl 102 entsprechend, mehr leitfähige Komponente zudosiert werden kann, so dass sich bei der Reaktion zwischen Leim und Härter eine ausreichende Abbindetemperatur einstellt. Auf diese Weise können die aus dem Infrarot-Scan 100 gemäß Figuren 7, 9 und 11 erhaltenen Informationen unmittelbar in den Herstellprozess 10 gemäß der Darstellung in Figur 1 rückgeführt werden, um nachfolgende Herstellprozesse zu verbessern.

## Patentansprüche

1. Verfahren zur Beeinflussung und Regelung eines Verleimungsprozesses (10) mit nachfolgenden Verfahrensschritten:
a) Vorgabe von Leimgangdaten (12) an einen Leimassistenten oder eine Steuerungsvorrichtung (16),
b) Übermittlung (20) ermittelter Parameter für eine Beleimung (32) an eine Auftragsanlage (22),
c) Vornahme einer Beleimung (32) mittels eines Härterauftrags (26) und/oder eines Leimauftrages (24) von welchem eine oder beide Komponenten eine elektrische Leitfähigkeit aufweisen, auf Lamellen (37) mindestens eines Brettschichtholzbauteils (36),
d) taktweise oder kontinuierliche Verpressung mindestens eines gemäß c) behandelten Brettschichtholzbauteils (36) in einer Pressvorrichtung (34),
e) Thermographie-Bild-Erfassung von Einzelfugen (108) durch eine Thermographie-Kamera (38) und Auswertung daraus bestimmter Fugentemperatur-Diagramme (110) und
f) Detektion fehlerhafter Fugen (114, 120) innerhalb des Fugentemperatur-Diagrammes (110) und Übermittlung von Korrekturdaten (42, 44) an den Leimassistenten (16) oder die Steuerungsvorrichtung (16), und/oder die Auftragsanlage (22) und/oder die Pressvorrichtung (34) und/oder den Bediener zur Beeinflussung der Beleimungs- und/oder Prozessparameter, **dadurch gekennzeichnet, dass** lokal eine Dosierung der elektrisch leitfähigen Komponente, Leim und/oder Härter zur Energiezufuhr dort erhöht wird, wo eine zu niedrige Fugentemperatur gemäß des Fugentemperaturdiagrammes (110) ermittelt wurde.

2. Verfahren gemäß Anspruch 1, **dadurch gekennzeichnet, dass** gemäß Verfahrensschritt f) eine Durchschnittsfugentemperatur jeder Einzelfuge (108) in einem Infrarot-Scan (100) ermittelt wird.

3. Verfahren gemäß einem der vorhergehenden Ansprüche, **dadurch gekennzeichnet, dass** im Fugentemperatur-Diagramm (110) Einzelfugen (108), deren Durchschnittstemperatur unterhalb der empfohlenen Durchschnittsfugentemperatur liegt, als Fehlfugen (114) kenntlich gemacht und lokalisiert werden.

4. Verfahren gemäß einem der vorhergehenden Ansprüche, **dadurch gekennzeichnet, dass** im Fugentemperatur-Diagramm (110) Einzelfugen (108), die Unterbrechungen (120) aufweisen, als Fehlfugen (114) kenntlich gemacht und lokalisiert werden.

5. Verfahren gemäß einem der vorhergehenden Ansprüche, **dadurch gekennzeichnet, dass** unzulässig hohe Messwertschwankungen, bezogen auf die Temperaturen innerhalb einer Einzelfuge (108), kenntlich macht und lokalisiert werden.

6. Verfahren gemäß dem vorhergehenden Anspruch, **dadurch gekennzeichnet, dass** an einer kritischen, frei wählbaren Stelle von der Oberflächentemperatur auf eine zu erwartende Bohrlochtemperatur zurückgerechnet wird, und wobei bei einer unzulässig tiefen Temperatur eine Kenntlichmachung erfolgt.

7. Verfahren gemäß Anspruch 1, **dadurch gekennzeichnet, dass** die leitfähige Komponente hinsichtlich deren Auftragsmenge abhängig von der Fugenanzahl (86, 102) lokal in Bezug auf die betreffende Stelle im Bauteil verändert wird und/oder die Auftragsmenge der leitfähigen Komponente über die Gesamtauftragsmenge der Leimflotte oder das Mischungsverhältnis mit der anderen Komponente eingestellt wird.

8. Verfahren gemäß einem der vorhergehenden Ansprüche, **dadurch gekennzeichnet, dass** bei Durchlaufpressen (88) eine automatische Regelung des Vorschubs (50), welche die Prozesszeit bestimmt, abhängig von den Korrekturdaten (42, 44) vorgenommen wird.

9. Verfahren gemäß einem der vorhergehenden Ansprüche **dadurch gekennzeichnet, dass** bei Taktpressen (90) eine automatische Regelung der Presszeit abhängig von den Korrekturdaten (42, 44) vorgenommen wird.

10. Verfahren gemäß einem der vorhergehenden Ansprüche **dadurch gekennzeichnet, dass** die Schwankung der durchschnittlichen Fugentemperaturen der Einzelfugen (108) untereinander als Grundlage für mathematische Funktionen dienen, nach welchen die elektrisch leitfähige Komponente dosiert wird.

## Claims

1. Method for influencing and controlling a glueing process (10) comprising the following method steps:
a) specifying glueing operation data (12) to a glueing assistant or a control device (16),
b) transmitting (20) determined parameters for a glueing (32) to an applicator (22),
c) performing a glueing (32) by means of application of a catalyst (26) and/or an application of a glue (24), of which one or both components have an electrical conductivity, to laminations (37) of at least one glued laminated timber structural element (36),
d) intermittently or continuously pressing at least one glued laminated timber structural element (36) treated according to c) in a pressing device (34),
e) taking a thermographic image of individual joints (108) by a thermographic camera (38) and evaluating joint-temperature diagrams (110) determined therefrom and
f) detecting defective joints (114, 120) within the joint-temperature diagram (110) and transmitting correction data (42, 44) to the glueing assistant (16) or the control device (16), and/or the applicator (22) and/or the pressing device (34) and/or the operator for influencing the glueing and/or process parameters, **characterized in that** a metering of the electrically conductive component, glue and/or catalyst with respect to the energy supply is increased locally wherever too low a joint temperature was determined according to the joint-temperature diagram (110).

2. Method according to Claim 1, **characterized in that**, according to method step f), an average joint temperature of each individual joint (108) is determined in an infrared scan (100).

3. Method according to either of the preceding claims, **characterized in that** in the joint temperature diagram (110) individual joints (108) of which the average temperature lies below the recommended average joint temperature are identified as defective joints (114) and localized.

4. Method according to one of the preceding claims, **characterized in that** in the joint temperature diagram (110) individual joints (108) that have interruptions (120) are identified as defective joints (114) and localized.

5. Method according to one of the preceding claims, **characterized in that** inadmissibly high measured-value fluctuations with respect to the temperatures within an individual joint (108) are identified and localized.

6. Method according to the preceding claim, **characterized in that**, at a critical, freely selectable location, the surface temperature is used to calculate back to a likely drill-hole temperature, and wherein, if there is an inadmissibly low temperature, an identification is performed.

7. Method according to Claim 1, **characterized in that**, depending on the number of joints (86, 102), the conductive component is changed with regard to the applied amount thereof locally with respect to the location concerned in the structural element and/or the applied amount of the conductive component is set by way of the overall applied amount of the glue mix or the mixing ratio with the other component.

8. Method according to one of the preceding claims, **characterized in that**, in the case of continuous presses (88), an automatic control of the advancement (50), which determines the process time, is performed on the basis of the correction data (42, 44).

9. Method according to one of the preceding claims, **characterized in that**, in the case of intermittent presses (90), an automatic control of the pressing time is performed on the basis of the correction data (42, 44).

10. Method according to one of the preceding claims, **characterized in that** the fluctuation of the average joint temperatures of the individual joints (108) in relation to one another serve as a basis for mathematical functions according to which the electrically conductive component is metered.

## Revendications

1. Procédé destiné à influencer et réguler un processus de collage (10), le procédé comprenant les étapes suivantes :
a) préréglage des données de processus de collage (12) au niveau d'un assistant de collage ou d'un dispositif de commande (16),
b) transmission (20) de paramètres déterminés pour un collage (32) à un système d'application (22),
c) réalisation d'un collage (32) au moyen d'une application de durcisseur (26) et/ou d'une colle (24), dont l'un des composants, ou les deux, ont une conductivité électrique, sur des lamelles (37) d'au moins un composant en bois lamellé collé (36),
d) pressage intermittent ou continu d'au moins un composant en bois lamellé collé (36), traité conformément à c), dans un dispositif de pressage (34),
e) capture d'image thermographique de joints individuels (108) par une caméra thermographique (38) et évaluation de diagrammes de température de joint (110) déterminés à partir de ladite caméra et
f) détection de joints défectueux (114, 120) dans le diagramme de température de joint (110) et transmission de données de correction (42, 44) à l'assistant de collage (16) ou au dispositif de commande (16), et/ou au système d'application (22) et/ou au dispositif de pressage (34) et/ou à l'opérateur pour influencer les paramètres de collage et/ou de processus, **caractérisé en ce que**
un dosage du composant électriquement conducteur, de la colle et/ou du durcisseur est augmenté localement pour l'apport en énergie là où une température de joint trop basse a été déterminée selon le diagramme de température de joint (110).

2. Procédé selon la revendication 1, **caractérisé en ce que**, selon l'étape de procédé f), la température de joint moyenne de chaque joint individuel (108) est déterminée dans un balayage infrarouge (100).

3. Procédé selon l'une des revendications précédentes, **caractérisé en ce que** des joints individuels (108) dont la température moyenne est inférieure à la température de joint moyenne recommandée, sont identifiés comme étant des joints défectueux (114) et localisés dans le diagramme de température de joint (110).

4. Procédé selon l'une des revendications précédentes, **caractérisé en ce que** des joints individuels (108), qui comportent des interruptions (120), sont identifiés comme étant des joints défectueux (114) et localisés dans le diagramme de température de joint (110).

5. Procédé selon l'une des revendications précédentes, **caractérisé en ce que** des fluctuations de valeur de mesure trop élevées, basées sur les températures à l'intérieur d'un joint individuel (108), sont identifiées et localisées.

6. Procédé selon la revendication précédente, **caractérisé en ce qu'**à un point critique et arbitraire de la température de surface un calcul est effectué en fonction de la température de forure attendue et dans lequel une identification est effectuée à une température basse inacceptable.

7. Procédé selon la revendication 1, **caractérisé en ce que** le composant conducteur est modifié, en termes de quantité à appliquer, localement par rapport au point concerné dans le composant en fonction du nombre de joints (86, 102) et/ou la quantité de composant conducteur à appliquer est réglée sur la quantité totale à appliquer de la colle ou sur le rapport de mélange avec l'autre composant.

8. Procédé selon l'une des revendications précédentes, **caractérisé en ce que**, dans les presses continues (88), une régulation automatique de l'avancement (50), qui détermine le temps de processus, est effectuée en fonction des données de correction (42, 44).

9. Procédé selon l'une des revendications précédentes, **caractérisé en ce que** dans les presses à alimentation intermittente (90), une régulation automatique du temps de pressage est effectuée en fonction des données de correction (42, 44).

10. Procédé selon l'une des revendications précédentes, **caractérisé en ce que** la fluctuation des températures moyennes des joints individuels (108) les unes par rapport aux autres servent de base à des fonctions mathématiques permettant le dosage du composant électriquement conducteur.
